(19) Europäisches Patentamt
European Patent Office
Office européen des brevets

(11) Veröffentlichungsnummer : **0 002 510**
**B1**

(12) # EUROPÄISCHE PATENTSCHRIFT

(45) Veröffentlichungstag der Patentschrift : **11.02.81**

(51) Int. Cl.³ : **C 07 C 69/14**, C 07 C 67/08, C 07 C 67/14, C 11 B 9/00

(21) Anmeldenummer : **78101620.9**

(22) Anmeldetag : **11.12.78**

(54) **Cyclohexane, Verfahren zu deren Herstellung, deren Verwendung und diese enthaltende Kompositionen.**

(30) Priorität 12.12.77 LU 78670
30.10.78 CH 11175

(43) Veröffentlichungstag der Anmeldung : **27.06.79 (Patentblatt 79/13)**

(45) Bekanntmachung des Hinweises auf die Patenterteilung : **11.02.81 Patentblatt 81/06**

(84) Benannte Vertragsstaaten : **BE CH DE FR GB IT NL**

(56) Entgegenhaltungen :
**DE - A - 2 317 000**
**BULLETIN DE LA SOCIETE CHIMIQUE DE FRANCE, 1970, Nr. 6, Paris (FR)**
**Y. HENG SUEN et al. « Préparation du t-butyl-2 méthylène cyclohexane des cis et trans méthyl-1 t-butyl-2 cyclohexanols et de leurs produits de déshydratation », Seiten 2270-2272**

(73) Patentinhaber : **L. Givaudan & Cie Société Anonyme**
**Patentdienst Postfach**
**CH-4002 Basel (CH)**

(72) Erfinder : **Heimlinger, Daniel, Dr.**
**Tichelrütistrasse 18**
**CH-8044 Gockhausen (CH)**
Erfinder : **Naegeli, Peter, Dr.**
**Vordere Höhenstrasse 31**
**CH-5430 Wettingen (CH)**

(74) Vertreter : **Lederer, Franz, Dr. et al**
**Patentanwälte Lederer, Meyer Lucile-Grahn-Strasse 22**
**D-8000 München 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

**EP 0 002 510 B1**

# Cyclohexane, Verfahren zu deren Herstellung, deren Verwendung und diese enthaltende Kompositionen

Die Erfindung betrifft neue Riechstoffe, nämlich Verbindungen der allgemeinen Formel

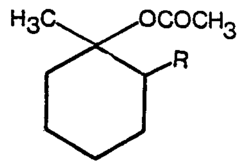

I

worin R sek. Butyl, tert. Butyl oder Cyclohexyl darstellt.

Die Erfindung betrifft auch ein Verfahren zur Herstellung dieser neuen Verbindungen der Formel I. Es ist dadurch gekennzeichnet, dass man eine Verbindung der allgemeinen Formel

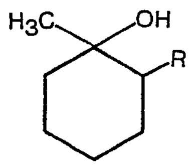

II

worin R obige Bedeutung, besitzt, mit einem Acetylierungsmittel behandelt.

Die Veresterung der Alkohole II wird bevorzugt unter Verwendung der Acetylhalogenide durchgeführt. Man arbeitet zweckmässigerweise in Anwesenheit von tertiären Aminen, wie Pyridin oder Dimethylanilin, bzw. in Anwesenheit von Alkali- oder Erdalkaliacetaten, oder anderer organischer Basen. Bevorzugt sind Pyridin und Dimethylanilin.

Man kann aber auch unter Verwendung des Säureanhydrides arbeiten ; die Acetate I sind so durch Umsetzung von II mit Essigsäureanhydrid bei Temperaturen zwischen 0-30 °C und Reaktionszeiten von 2-5 Tagen zugänglich, wobei katalytische Mengen von Phosphorsäure oder auch irgend eines anderen üblichen Katalysators zur Verwendung gelangen ; die Veresterung kann aber auch durchgeführt werden unter Zuhilfenahme des Säureanhydrides bei Rückflusstemperatur (oder allgemein zwischen ungefähr 20-140 °C), gegebenenfalls unter Zusatz eines Alkalimetallsalzes der Essigsäure ; die Reaktionsdauer kann z.B. 2-30 Stunden betragen. Die Reaktionsdauer variiert selbstverständlich je nach der Natur der eingesetzten Reaktionspartner. Die Umsetzungen können in jedem Fall unter Zuhilfenahme von Lösungsmitteln, wie Aromaten, z.B. Benzol, Toluol, oder Aliphaten, z.B. Hexan oder Heptan, oder chlorierten Aliphaten durchgeführt werden. Schliesslich kann auch ein tertiäres Amin als Lösungsmittel dienen.

Die Ester I werden zweckmässig durch Destillation unter vermindertem Druck gereinigt ; sie stellen farblose bis leicht gelblich gefärbte Flüssigkeiten oder niedrigschmelzende kristalline Substanzen dar, sind unlöslich in Wasser, aber löslich in organischen Lösungsmitteln, wie z.B. Alkoholen, Aethern, Ketonen, Estern, Kohlenwasserstoffen und halogenierten Kohlenwasserstoffen.

Die als Ausgangsmaterialien für das erfindungsgemässe Verfahren dienenden Verbindungen der allgemeinen Formel II können dadurch erhalten werden, dass man eine Verbindung der allgemeinen Formel

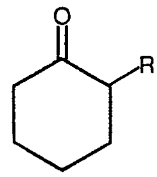

III

worin R obige Bedeutung, besitzt, mit einer organometallischen Verbindung der allgemeinen Formel

$$MCH_3 \quad IV$$

worin M ein Alkalimetall oder die Gruppierung MgX, worin X Halogen ist, darstellt, umsetzt.

Bevorzugt ist die Reaktion des Ketons mit der entsprechenden Grignardverbindung. In bezug auf Lösungsmittel, Reaktionstemperatur und Mengen der umzusetzenden Reaktionspartner kann man sich anhand der Literatur orientieren.

Im Falle der Grignardverbindung also z.B. in Lösungsmitteln wie Aethern, z.B. Diäthyläther, Tetrahydrofuran, etc. bzw. in Kohlenwasserstoffen unter Zusatz von Aethern und bei Temperaturen von (Vorzugsweise) 0 °C bis 50 °C arbeiten.

Man wird normalerweise die Grignardverbindung und das Keton in stöchiometrischen Mengen einsetzen, obschon z.B. ein Ueberschuss von bis zu 20 % an Grignardverbindung oder Keton keinen nachteiligen Einfluss auf den Ablauf der Reaktion hat.

Im Falle der Methylmetallverbindungen arbeitet man zweckmässigerweise mit der üblichen Lösung von Methyllithium in Diäthyläther in einem Temperaturbereich von − 75 °C bis ca. 35 °C, vorzugsweise bei − 35 °C bis Raumtemperatur.

Die Alkohole II können z.B. durch Destillation unter vermindertem Druck (ungefähr 13,3-700 Pa) gereinigt werden. Sie stellen farblose bis schwach gelblich gefärbte Flüssigkeiten dar, sind unlöslich in Wasser, aber löslich in Alkoholen, Ketonen, Aethern, Estern und Kohlenwasserstoffen.

Die Ester I der vorliegenden Erfindung liegen als cis-trans-Stereoisomere vor, und die Geruchseigenschaften dieser Isomeren sind etwas verschieden. Die Isomeren können nach den üblichen Methoden, die für die Auftrennung von solchen Stereoisomeren bekannt sind, aufgetrennt werden. Es hat sich gezeigt, dass fraktionierte Destillation der entsprechenden Alkohole unter vermindertem Druck zur Auftrennung der Isomeren führt, welche ihrerseits wiederum nach den oben beschriebenen Methoden zu stereoisomeren

Estern umgesetzt werden können. Für die Verwendung der neuen Verbindungen I zu parfümistischen Zwecken ist jedoch diese Auftrennung normalerweise nicht nötig.

Die Ester I weisen besondere organoleptische Eigenschaften auf, auf Grund derer sie sich vorzüglich als Riechstoffe eignen.

Die Erfindung betrifft demgemäss auch die Verwendung von I als Riechstoffe, und Riechstoffkompositionen, die durch einen Gehalt an diesen Estern I gekennzeichnet sind.

Die erfindungsgemäss als Riechstoffe verwendeten Verbindungen I zeichnen sich insbesondere durch interessante Ambranuancen aus. Von besonderem Interesse ist, dass diese Ambranoten bereits in den Kopfnoten der Riechstoffkompositionen auftreten. Da andererseits die Verbindungen I nicht zu flüchtig sind, bleiben die Nuancen auch in der Fondnote erhalten.

Die Verbindungen können demgemäss beispielsweise zur Parfümierung von Produkten, wie Kosmetika (Seifen, Waschmitteln, Detergentien, Raucherartikeln, Mundwässern, Desodorantien, Shampoos, Lotions, Salben, Pudern, Eau de Toilette, Eau de Cologne, Extraits, etc.), dienen, wobei die Verbindungen I vorzugsweise nicht allein, sondern in Form von Kompositionen mit andern Riechstoffen eingesetzt werden.

Als Riechstoffe eignen sich die Verbindungen I auf Grund ihres hohen harmonischen Einfügungsvermögens, insbesondere in Kombination mit einer Reihe von natürlichen und synthetischen Riechstoffen.

Die erfindungsgemässen Verbindungen harmonieren besonders gut mit folgenden Klassen von chemischen Substanzen bzw. mit folgenden natürlichen Gemischen :

mit Bergamotteöl, Hysopöl, Patchouliblätteröl, Patchouliöl, Vetiveröl, Cedernholzöl, Cedernblätteröl, Galbanumöl, Angelikasamenöl, Petitgrainöl, Sandelholzöl, Eichenmoos, Labdanumöl, Corianderöl, Grapefruitöl, Castoreum abs., Vetiverol, Vetivenylacetat, nat. Moschus und Zibetextrakten sowie Ambregrisinfusion,

mit Alkoholen, wie z.B. Aethanol, Propylenglykol, Phenyläthyl- und Phenylpropylalkohol, Geraniol, Nerol, Citronellol, Linalool, Santalol, Farnesol, Terpineol, Phenyldimethylcarbinyol,

mit Ketonen, wie Jasmon, Dihydrojasmon etc., Jononen, Ironen, Raldeinen® (Methyljononen), Allyljononen, Muscon, Exalton, Zibeton, Versalide® (7-Acetyl-1,1,4,4-tetramethyl-7-äthyl-1,2,3,4-tetralin), β-Mercaptopulegon,

mit Aldehyden und ihren Acetalen, wie Citral, Citronellal, Hydroxycitronellal, Lilial, Cyclamenaldehyd, α-Hexylzimtaldehyd, Heliotropin, Vanillin, Phenylacetaldehyd, Anisaldehyd,

mit Aethern, wie Theaspiran, 1-Methylcyclododecylmethyläther, Cedryläther, α- und β-Ionyl- und Dihydrojonyläther, Gujacyläther, Oestragol, Anethol,

mit Kohlenwasserstoffen, wie Limonen, Caren, α- und β-Pinen, Myrcen, Ocimen, Farnesen,

mit phenolischen Körpern, wie Eugenol, Isoeugenol, Chavicol,

mit Estern, wie Linalylacetat, Benzylacetat, Amylsalicylat, Zimtsäureestern, Benzylsalicylat, Methyldihydrojasmonat, Allylphenoxyacetat, Formylacetat, Styrallylacetat, Phenyläthylphenylacetat,

mit Lactonen, wie α-Nonyllacton, Jasminlacton, Massoialacton, Oxahexadecanolid, Thibetolid, Aethylenbrassilat, Cumarinen, oder auch

mit stickstoffhaltigen Körpern, wie Pyrazinen, z.B. 2,5-Dimethylpyrazin, Ambrettemoschus, z.B. Ketonmoschus.

Die unter Verwendung von I hergestellten Riechstoffkompositionen bestechen insbesondere auch durch die ausserordentliche Strahlungskraft, Natürlichkeit und Lebhaftigkeit.

Die Konzentration der Verbindungen I kann je nach dem Verwendungszweck innerhalb weiter Grenzen variieren, beispielsweise zwischen etwa 0,5 (Detergentien) und etwa 10 Gew. % (alkoholische Lösungen). In Parfümbasen bzw. Konzentraten können die Konzentrationen selbstverständlich auch höher liegen. Die Parfümbasen können in üblicher Weise zur Parfümierung von Eaux de Cologne, Eaux de Toilette, Lotionen, Crèmes, Shampoos, Seifen und Detergentien, etc. verwendet werden.

Bei niedrigen Dosierungen (z.B. 0,5-2 %) von I kann bereits eine deutliche Zunahme der Strahlungskraft festgestellt werden, ohne dass der Grundcharakter der Komposition wesentlich verändert wird. Bei höheren Dosierungen (z.B. 10-30 %) tritt zusätzlich eine den olfaktischen Eigenschaften der verwendeten Verbindung entsprechende Modifizierung ein.

Beispiel 1

Zu einer Lösung von 576 g rohem 1-Methyl-2-cyclohexylcyclohexanol in 750 ml N,N-Dimethylanilin tropft man bei 0 °C unter Rühren eine Mischung von 360 ml Acetylchlorid und 153 ml Acetanhydrid zu. Man lässt das Gemisch zunächst Raumtemperatur annehmen, danach erhitzt man es während 36 Stunden auf 40 °C. Das Reaktionsgemisch wird hierauf auf Eis gegossen, mit 1 Liter Aether verdünnt, die organische Phase abgetrennt und am Rotavapor (Feinvakuum) eingeengt. Dann wird in 2 Litern Aether aufgenommen, dreimal mit je 200 ml kalter 2 n HCl, darauf mit gesättigter Bicarbonatlösung und Wasser bis zur Neutralität gewaschen, mit wasserfreiem Mg $SO_4$ getrocknet und unter Vakuum eingedampft. Man erhält 646 g rohes 1-Acetoxy-1-methyl-2-cyclohexyl-cyclohexan.

Durch Umkristallisation aus Methanol bei − 70 °C [oder aus Aethanol bei − 10 °C] erhält man 400 g eines Reinstkristallisates, Smp. 35 °C ; Geruch : diffusive Ambranote, Moschus, Holz.

Mikroanalyse : $C_{gef}$ 75,57 % $C_{ber}$ 75,58 %
$H_{gef}$ 11,16 % $H_{ber}$ 10,99 %

IR (Film) : *2960,* 2900, 2700, *1735,* 1450, 1360, 1270, *1235,* 1190, 1150, 1130, 1015,

982, 950, 925, 900, 860, 794, 740 $cm^{-1}$.

NMR ($CDCl_3$ + TMS) : δ = 3,1-2,4 ppm (m für 2H) ;
δ = 1,98 ppm (s, 3H) ;
δ = 1,54 ppm (s, 3H).

MS : m/e : 196 ($M^+ - CH_2CO$), 178 ($M^+$ – ACOH), 163, 149, 136, 121, 109, 97, 81, 71, 67, 55, *43*.

Beispiel 2

Zu einer gerührten Lösung von 2,615 kg des 1-Methyl-2-sek. butyl-cyclohexanols in 3,6 kg N,N-Dimethylanilin tropft man bei 0 °C eine Mischung von 1,77 kg Acetylchlorid und 0,75 kg Acetanhydrid. Man lässt das Gemisch Raumtemperatur annehmen und rührt es 18 Stunden bei 40 °C, worauf es langsam auf 6 kg Eis gegossen und mit 3 Litern Hexan verdünnt wird. Die wässrige Phase wird mit 1 Liter Hexan nachextrahiert. Die vereinigte organische Phase wird wie folgt gewaschen :

1 mal mit 1 Liter 2 n HCl + 1 kg Eis
2 mal mit 2 Litern 2 n HCl
1 mal mit 1 Liter 2 n HCl
2 mal mit 2 Litern Wasser
2 mal mit 1 Liter ges. $NaHCO_3$
1 mal mit 1 Liter Wasser ; nun wird die organische Phase mit 0,2 kg wasserfreiem $Na_2SO_4$ getrocknet und eingedampft.

Beim Rohprodukt handelt es sich um 3,04 kg 1-Acetoxy-1-methyl-2-sek.-butyl-cyclohexan.
Gehalt an Acetylverbindung > 85 % (Gaschromatogramm).

Durch Fraktionierung an einer 1 m-Füllkörperkolonne (Raschigringe 7 m/m) erhält man 2,23 kg olfaktisch einwandfreies Produkt : Geruch : Ambra, holzig, animalisch warm, tabakartig. Sdp : 39 °C/~ 0.133 Pa ;
IR (Film) : *2950*, 2880, *1735*, 1462, 1450, 1375, 1365, 1270, *1240*, 1230, 1190, 1155, 1020, 945, 860 $cm^{-1}$.

NMR ($CDCl_3$ + TMS) : δ = 3,1-2,4 ppm (m, 2H) ;
δ = 1,98 ppm (s, 3H) ;
δ = 1,53 ppm und 1,50 ppm (s, 3H) ;
δ = 0,96 bis 0,82 ppm (Dubletts und Tripletts für isomere sek. und prim. Methylgruppen für 6H).

MS : m/e : 170 ($M^+ - CH_2CO$), 152 ($M^+$ – ACOH), 141, 123, 110, 95, 81/82, 71, 67, 55, *43*.

Beispiel 3

Zu einer Lösung von 20 g des 1-Methyl-1-hydroxy-2-t-butyl-cyclohexans in 41,6 ml N,N-Dimethylanilin tropft man bei 0 °C ein Gemisch von 16 ml Acetylchlorid und 8 ml Acetanhydrid. Man lässt das Reaktionsgemisch Raumtemperatur annehmen und rührt es 24 Stunden. Hierauf giesst man auf Eis und extrahiert mit Aether. Die organische Schicht wird anschliessend mit 2N Salzsäure und gesättigtem Natriumbicarbonat gewaschen und über Natriumsulfat getrocknet. Die organische Phase wird eingeengt. Das Rohgemisch enthält (Gaschromatogramm) 32 % 2-t-Butylcyclohexanon, 20,4 % 1-Methyl-1-hydroxy-2-t-butylcyclohexan und 47,5 % 1-Acetoxy-1-methyl-2-t-butylcyclohexan. Durch Destillieren bei 26.6 Pa erhält man neben weniger reinen Fraktionen 2 g 98 % iges 1-Acetoxy-1-methyl-2-t-butyl-cyclohexan ; Siedepunkt 48-53 °C/26.6 Pa.

IR (liq) : 1730 $cm^{-1}$, 1360, 1260, 1230, 1150, 1180, 1015 $cm^{-1}$.

NMR ($CDCl_3$) : 1 ppm (9H) s ; 1,7 ppm (3H) s ; 2 ppm (3H) s.

MS : m/e : 212, 170, 137, 110, 96, 81, 71, 57, *43*.

Geruch : Ambraartig, holzig, cedrig, Patchouli, Vetiver.

In den folgenden Formulierungsbeispielen sind die unerwarteten organoleptischen Eigenschaften des neuen sek. Butylderivats anhand typischer Riechstoffkompositionen mit dem strukturell naheliegenden 1-Acetoxy-1-äthyl-2-sek. butyl-cyclohexan, im folgenden « Aethylderivat » bezeichnet, bzw. dem 1-Acetoxy-1-vinyl-2-sek. butyl-cyclohexan (« Vinylderivat ») (US-Patentschriften No. 3 769 330 und 3 852 219) verglichen.

Beispiel 4

Parfümerie-Base mit Salbeicharakter

| | Gewichtsteile |
|---|---|
| Methyl-dihydrojasmonat | 400 |
| Bergamotteöl | 200 |
| Hysopöl | 100 |
| Patchouliblätteröl | 100 |
| Allyljonon | 60 |
| Propylenglykol | 120 |
| | 980 |

Durch Zusatz von 2 % 1-Acetoxy-1-methyl-2-sek. butyl-cyclohexan wird in dieser würzig-grünen Komposition ein typischer warmer Salbeieffekt erzielt, der sich ausserordentlich gut für Herrenlinien eignet.

Im Gegensatz dazu erzeugt derselbe Zusatz an Aethylderivat diesen gewünschten Salbeieffekt nicht, die Komposition wirkt in diesem Fall trocken-holzig und honigartig, d.h. es resultiert bloss eine Hervorhebung der Hysopöl-Note.

Beispiel 5

Parfümerie-Komposition Richtung Tomate

| | Gewichtsteile |
|---|---|
| Bergamotteöl | 300 |
| Corps Cassis Givaudan®(8-Mercaptomenthon) | 320 |
| Grapefruitöl | 160 |
| Galbanumöl | 120 |
| Gamma-nonyl-lacton (10 % in Alkohol) | 60 |
| 2,5-Dimethyl-pyrazin | 10 |
| | 970 |

Durch einen Zusatz von 3 % 1-Acetoxy-1-methyl-2-sek. butyl-cyclohexan zu obiger Komposition resultiert in der fruchtig-grünen Base eine überraschende Tomatenblätternote, welche andererseits mit dem Aethylderivat nicht erreicht werden kann. Letztere Verbindung unterstreicht bloss den ursprünglichen fruchtig-citrusartigen Charakter der Base.

Beispiel 6

Parfümerie-Base mit grünem Charakter

| | Gewichtsteile |
|---|---|
| Isoraldein | 200 |
| Petitgrainöl | 100 |
| Galbanumöl | 100 |
| Musc 174® Givaudan (Oxa-4-pentadecanolid) | 100 |
| Bergamotteöl | 60 |
| Angelikasamenöl | 40 |
| Propylenglykol | 380 |
| | 980 |

Durch 2 % Zusatz an 1-Acetoxy-1-methyl-2-sek. butyl-cyclohexan in obiger Grünbase erzielt man einen ausgeprägten Ambracharakter, der durch dieselbe Dosierung an Aethylderivat nicht erreicht werden kann. Das Aethylderivat kann andererseits in der Komposition nicht höher dosiert werden, da sich somit deren Charakter ändern würde.

Beispiel 7

Cologne-Base

| | Gewichtsteile |
|---|---|
| Linalylacetat | 200 |
| Phenyläthylalkohol | 150 |
| Benzylsalicylat | 100 |
| Geraniol | 100 |
| Methyl-1-methylcyclododecyläther | 100 |
| Linalool | 50 |
| Aethylenbrassylat | 50 |
| Methyldihydrojasmonat | 30 |
| Hydroxycitronellal | 50 |
| α-Jonon | 30 |
| Lilial Givaudan® (4-tert. Butylphenyl-2-methyl-propanal) | 20 |
| Vetiverol | 20 |
| Santalol | 20 |
| Allyl-phenoxyacetat | 10 |
| Bornylacetat | 10 |
| Styrallylacetat | 10 |
| | 950 |

Setzt man obiger Colognebase 5 % 1-Acetoxy-1-methyl-2-sek. butyl-cyclohexan zu, so wirkt diese viel harmonischer, angenehm rosig und frischer.

Derselbe Zusatz an Aethylderivat hat eine Unterdrückung der Colognenote zur Folge.

Beispiel 8

Chypre-Komposition

| | Gewichtsteile |
|---|---|
| Madrox® (Methyl-1-methylcyclododecyläther) | 120 |
| Phenyläthyl-phenylacetat | 100 |
| Bergamotteöl | 100 |
| α-Jonon | 100 |
| a-Hexylzimtaldehyd | 100 |
| Benzylacetat | 50 |
| Vetivenylacetat | 80 |
| Citronellol | 70 |
| Linalool | 70 |
| Patchouliöl | 30 |
| Sandelholzöl | 30 |
| Eichenmoos absolut entfärbt | 30 |
| Eugenol | 30 |
| Keton-moschus | 30 |
| Ambrette-moschus | 20 |
| Labdanumöl französisch | 5 |
| Corianderöl | 5 |
| | 970 |

Gibt man zu obiger Chypre-Komposition 3 % 1-Acetoxy-1-methyl-2-sek. butyl-cyclohexan, erhält diese einen ausgeprägt warmen Ambracharakter, der der Komposition wesentlich mehr Diffusion *und* zugleich Haftfestigkeit verleiht.

Das Aethylderivat als Komponente lässt dieselbe Komposition bloss pudriger erscheinen.

Beispiel 9

Nelken-Base

| | Gewichtsteile |
|---|---|
| Eugenol | 300 |
| Isoeugenol | 175 |
| Benzylsalicylat | 100 |
| Amylsalicylat | 50 |
| Phenyläthyl-phenylacetat | 50 |
| Citronellol | 50 |
| α-Jonon | 50 |
| Hydroxycitronellal | 50 |
| α-Hexylzimtaldehyd | 50 |
| Benzylacetat | 50 |
| Terpineol | 30 |
| Heliotropin | 20 |
| | 975 |

Durch Zugabe von 0,5-1 % 1-Acetoxy-1-methyl-2-sek. butyl-cyclohexan wird bereits der gleiche Effekt wie mit der 5-fachen Menge Aethylderivat erreicht.

Beispiel 10

Parfümerie (Moos)-Base

| | Gewichtsteile |
|---|---|
| Hydroxycitronellal | 200 |
| Bornylacetat | 300 |
| Terpenylacetat | 180 |
| a-Hexylzimtaldehyd | 100 |
| Benzylacetat | 50 |
| Linalool | 40 |
| α-Jonon | 20 |
| Styrallylacetat | 20 |
| Zypressenöl | 20 |
| Rosmarinöl | 10 |
| C-12-Aldehyd (Laurinaldehyd) (10 % in Propylenglykol) | 10 |
| Lavandinöl | 10 |
| Eugenol | 10 |
| Citral | 6 |
| p-Menthan-8-thiol-3-on | 4 |
| | 980 |

Gibt man zu diesem frischen Wald-Duft 20 Teile 1-Acetoxy-1-methyl-2-sek. butyl-cyclohexan, so wirkt die erhaltene Base viel wärmer, holziger, sie zeigt mehr Volumen. Eine neue Note Richtung Chypre tritt auf.

Ein Zusatz von 20 Teilen des Vinylderivates erbringt andererseits keine Veränderung der ursprünglichen Base.

Beispiel 11

Parfümerie-Base (Richtung Fougère)

| | Gewichtsteile |
|---|---|
| Lavendelöl | 200 |
| Amylsalicylat | 200 |
| Cumarin | 100 |
| Baum-Moos (50 % in Propylenglykol) | 100 |
| Citronellol | 60 |
| Geraniol | 60 |
| Ambrette-Moschus | 60 |
| Bergamotteöl | 60 |
| α-Jonon | 20 |
| Vetiveröl | 20 |
| Sandelholzöl | 20 |
| α-Amylzimtaldehyd | 20 |
| Eugenol | 20 |
| Patchouliöl | 20 |
| | 960 |

Ein Zusatz von 40 Teilen 1-Acetoxy-1-methyl-2-sek. butyl-cyclohexan erbringt viel mehr Strahlungskraft und Volumen in der Komposition. Die Base wirkt viel wärmer, holziger und runder.

Andererseits hat ein Zusatz von 40 Teilen Vinylderivat keinerlei Verbesserung der Komposition zur Folge.

Beispiel 12

Parfümerie-Base (Richtung Herren-Cologne)

| | Gewichtsteile |
|---|---|
| Bergamotte synthetisch | 120 |
| Cedrylacetat | 100 |
| Zitronenöl synthetisch | 80 |
| α-Jonon | 80 |
| Weihrauchöl | 60 |
| Sandela® Givaudan (3-Isocamphyl-(5)-cyclohexanol) | 60 |
| Madrox® | 60 |
| Fixolide® Givaudan (1,1,2,4,4,7-Hexamethyl-6-acetyl-1,2,3,4-tetrahydronaphthalin) | 60 |
| Linalool | 60 |
| Hydroxycitronellal | 50 |
| Benzylacetat | 40 |
| cis-Jasmon | 10 |
| Galbanumöl | 10 |
| Styrallylacetat | 10 |
| Cumarin | 10 |
| Heliotropin | 10 |
| C-10-Aldehyd (10 % in Propylenglykol) | 10 |
| C-11-Aldehyd (10 % in Propylenglykol) | 10 |
| Beifussöl | 10 |
| Cyclal C® Givaudan (3,5-Dimethylcyclohex-3-en-1-ylcarboxaldehyd) | 10 |

| | |
|---|---|
| Castoreumöl synthetisch | 10 |
| Agrumex® Givaudan (o-tert.-Butylcyclohexylacetat) | 10 |
| Thymianöl weiss | 10 |
| Ciste Labdanumöl französisch | 10 |
| Nelkenöl rektifiziert | 10 |
| Estragol | 6 |
| Fructone® IFF (2-Methyl-1,3-dioxolan-2-äthylacetat) | 6 |
| Isobutylchinolin (10 % in Propylenglykol) | 8 |
| Geranylacetat | 20 |
| | 950 |

Gibt man zu obiger Base, welche sich bereits als solche für Herren-Linien eignet, 50 Teile 1-Acetoxy-1-methyl-2-sek. butyl-cyclohexan, so wirkt die neue Komposition wärmer, runder und voller. Es tritt nun eine sehr gesuchte, warmanimalische Note in Erscheinung.

Ein Zusatz von 5 Teilen des Vinylderivats hat keinen Effekt auf die Base.

Beispiel 13

A. Haftvermögen auf Textilien (Substantivität)

Die Substantivität von 1-Acetoxy-1-methyl-2-sek. butyl- cyclohexan a), vom Aethylderivat b) und vom Vinylderivat c) wurde durch Waschversuche unter verschiedenen Bedingungen untersucht.

| Waschtemperatur | |
|---|---|
| 10° | handgewaschene Wäsche |
| 60° | Automaten-Wäsche |
| 95° | Automaten-Wäsche |

Ein Panel von 10 Personen stellte fest, dass unter allen Bedingungen a) weitaus am besten abschnitt :

a) erwies sich auf der gewaschenen Wäsche als kräftiger, haftfester, und die so behandelten Textilien hinterliessen den typischen Eindruck sauberer Wäsche.

Ein Vergleich der Substantivität ergab folgendes Bild :

| Verbindung | Vergleichszahl für Substantivität |
|---|---|
| a) | 2 |
| b) | 0,5 |
| c) | 0,7 |

B. Verdünnungsreihen

Es wurden mit den Produkten a) b) und c) Verdünnungsreihen von 100 %, 10 %, 1 %, 0,1 % und 0,01 % erstellt. Es war so wiederum sofort ersichtlich, dass das Produkt a) auf dem Riechstreifen den bei weitem niedrigsten Geruchsschwellenwert aufwies : bei 0,1 und 0,01 % war nur noch a) wahrnehmbar.

C. Einarbeitung in Basen

vom Typ Fougère, Moos und Ligne masculine ergab, dass Konzentrationen von 2, 3 und 4 Gew. % a) vollauf genügten, um diese Basen zu veredeln, währenddem dies im Falle von b) und c) nicht der Fall war. In den letzten Fällen waren 5-10 % nötig, um denselben Effekt zu erzielen.

**Ansprüche**

1. Verbindungen der allgemeinen Formel

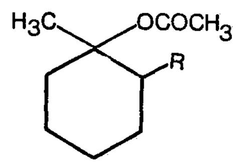

I

worin R sek. Butyl, tert. Butyl oder Cyclohexyl darstellt.

2. 1-Acetoxy-1-methyl-2-sek. butyl-cyclohexan.

3. 1-Acetoxy-1-methyl-2-tert. butyl-cyclohexan.

4. 1-Acetoxy-1-methyl-2-cyclohexyl-cyclohexan.

5. Verbindungen der allgemeinen Formel

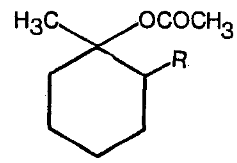

I

worin R sek. Butyl, tert. Butyl oder Cyclohexyl darstellt, als Riechstoffe.

6. 1-Acetoxy-1-methyl-2-sek. butyl-cyclohexan als Riechstoff.

7. Riechstoffkomposition, gekennzeichnet durch einen Gehalt an einer Verbindung der allgemeinen Formel

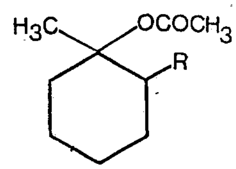

I

worin R sek. Butyl, tert. Butyl oder Cyclohexyl darstellt.

8. Riechstoffkomposition, gekennzeichnet durch einen Gehalt an 1-Acetoxy-1-methyl-2-sek. butyl-cyclohexan.

9. Verfahren zur Herstellung von Verbindungen der allgemeinen Formel

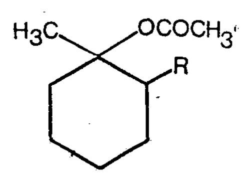

I

worin R sek. Butyl, tert. Butyl oder Cyclohexyl darstellt, dadurch gekennzeichnet, dass man eine Verbindung der allgemeinen Formel

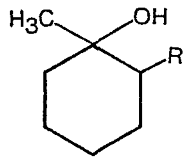

II

worin R obige Bedeutung besitzt, mit einem Acetylierungsmittel behandelt.

10. Verwendung von Verbindungen der allgemeinen Formel

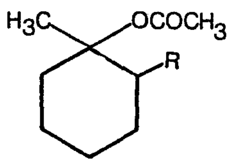

I

worin R sek. Butyl, tert. Butyl oder Cyclohexyl darstellt, als Riechstoffe.

11. Verwendung von 1-Acetoxy-1-methyl-2-sek. butyl-cyclohexan gemäss Anspruch 10.

**Claims**

1. Compounds of the general formula

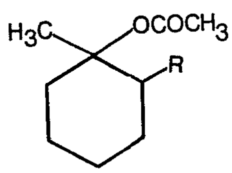

(I)

wherein R represents sec. butyl, tert. butyl or cyclohexyl.

2. 1-Acetoxy-1-methyl-2-sec. butyl-cyclohexane.

3. 1-Acetoxy-1-methyl-2-tert. butyl-cyclohexane.

4. 1-Acetoxy-1-methyl-2-cyclohexyl-cyclohexane.

5. Compounds of the general formula

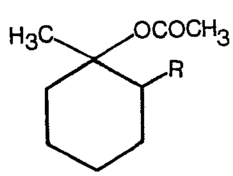

(I)

wherein R represents sec. butyl, tert. butyl or cyclohexyl, when used as odorants.

6. 1-Acetoxy-1-methyl-2-sec. butyl-cyclohexane when used as odorant.

7. An odorant composition characterized in that it contains a compound of the general formula

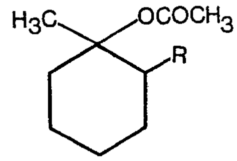

(I)

wherein R represents sec. butyl, tert. butyl or cyclohexyl.

8. An odorant composition characterized in that it contains 1-acetoxy-1-methyl-2-sec. butyl-cyclohexane.

9. Process for the manufacture of a compound of the general formula

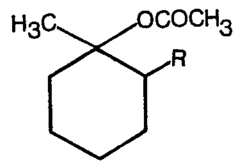

(I)

wherein R represents sec. butyl, tert. butyl or cyclohexyl, characterized in that a compound of the general formula

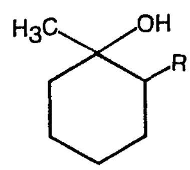

(II)

wherein R has the above significance, is treated with an acetylating agent.

10. The use of compounds of the general formula

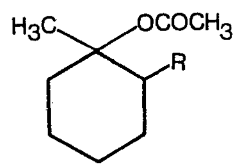

(I)

wherein R represents sec. butyl, tert. butyl or cyclohexyl, as odorants.

11. The use of 1-acetoxy-1-methyl-2-sec. butyl-cyclohexane according to claim 10.

**Revendications**

1. Composés de formule générale

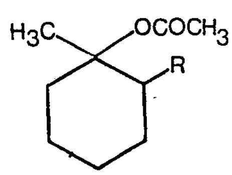

I

dans laquelle R représente un groupe sec. butyle, tert.-butyle ou cyclohexyle.

2. 1-Acétoxy-1-méthyl-2-sec. butyl-cyclohexane.

3. 1-Acétoxy-1-méthyl-2-tert.-butyl-cyclohexane.

4. 1-Acétoxy-1-méthyl-2-cyclohexyl-cyclohexane.

5. Composés de formule générale

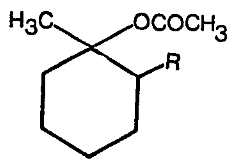

I

dans laquelle R représente un groupe sec. butyle, tert.-butyle ou cyclohexyle, comme odorants.

6. 1-Acétoxy-1-méthyl-2-sec. butyl-cyclohexane comme odorant.

7. Composition odorante, caractérisée par une teneur en un composé de formule générale

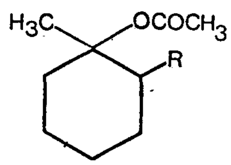

I

dans laquelle R représente un groupe sec. butyle, tert.-butyle ou cyclohexyle.

8. Composition odorante, caractérisée par une teneur en 1-acétoxy-1-méthyl-2-sec. butyl-cyclohexane.

9. Procédé pour la préparation de composés de formule générale

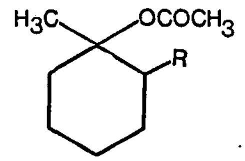

I

dans laquelle R représente un groupe sec. butyle, tert.-butyle ou cyclohexyle, caractérisé en ce qu'on traite un composé de formule générale

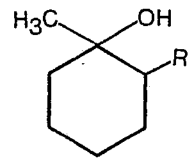

II

dans laquelle R a la signification ci-dessus, avec un agent d'acétylation.

10. Utilisation de composés de formule générale

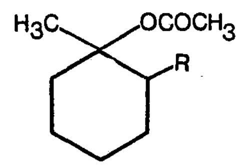

I

dans laquelle R représente un groupe sec. butyle, tert.-butyle ou cyclohexyle, comme odorants.

11. Utilisation de 1-acétoxy-1-méthyl-2-sec. butyl-cyclohexane selon la revendication 10.